# EUROPEAN PATENT APPLICATION

(11) **EP 1 632 215 A1**
(43) Date of publication of application: **08.03.2006**
(21) Application number: 05017206.3
(22) Date of filing: 08.08.2005
(51) Int. Cl.: A61K 8/36, A61K 8/34, A61Q 15/00

(54) **Liquid deodorant or antiperspirant preparations containing unsaturated fatty acids**

(30) Priority: 09.08.2004 JP 2004232351
(71) Applicant: KAO CORPORATION, Chuo-ku, Tokyo (JP)
(72) Inventor: Fuji, Akira, 2-1-3, Bunka Sumida-ku Tokyo (JP); Hagura, Toyoki, 2-1-3, Bunka Sumida-ku Tokyo (JP)
(74) Representative: HOFFMANN EITLE

(57) **Abstract**

A liquid deodorant preparation containing (A) an antiperspirant and/or an antimicrobial agent, (B) an unsaturated fatty acid, (C) an antioxidant, and (D) an aqueous medium. The liquid deodorant preparation is excellent in a silky skin feeling after application, easy to handle, and less irritant to human skin.

## Description

### BACKGROUND OF THE INVENTION

### 1. Field of the Invention

The present invention relates to liquid deodorant preparations.

### 2. Description of the Prior Art

Sweat secreted from perspiratory glands such as ecerine gland and apocrine gland contains amino acids, neutral fats, fatty acids, cholesterol, etc. These compounds are decomposed into malodor substances by the action of resident skin flora, to form body odors such as perspirant odor, armpit odor and foot odor. The formation of body odors are prevented generally by inhibiting the secretion of sweat by an antiperspirant having an astringent action on skin proteins; preventing the multiplication or growth of resident skin flora by an antimicrobial agent; changing lower fatty acids, which arc thought to be one of causes for armpit odor and foot odor, into their metal salts; or masking malodors by a fragrance or cologne water. The cosmetic products used in these methods are generally referred to as deodorant preparations.

Deodorant preparations are utilized in various application forms such as liquids, sprays, roll-ons, aerosols, solids and powders, with liquid preparations being suporior to spray preparations because a necessary amount can be applied without wasting. However, since human skin is wet in summer, a liquid deodorant preparation is difficult to dry after application to human skin, failing to provide a refreshing feeling immediately.

A technique directed to the drying behavior of a liquid deodorant preparation containing an antimicrobial agent, a powder and an aqueous medium has been proposed in JP 2001-302482A. However, a sufficient result has not been obtained yet.

### SUMMARY OF THE INVENTION

The inventors have found that liquid deodorant preparations incorporated with a small amount of unsaturated fatty acid quickly dry to provide a silky feeling immediately after application even when human skin is wet, and are easy to use, less irritant to human skin and excellent in deodorant effects.

Thus, the present invention provides a liquid deodorant preparation containing (A) an antiperspirant and/or an antimicrobial agent, (B) an unsaturated fatty acid, (C) an antioxidant, and (D) an aqueous medium.

### DETAILED DESCRIPTION OF THE INVENTION

The component (A) of the liquid deodorant prerparations of the present invention is an antiperspirant and/or an antimicrobial agent.

Examples of the antiperspirants include aluminum chlorohydrate, aluminum chloride, aluminum sulfate, basic aluminum bromide, aluminum phenolsulfonate, tannic acid, aluminum naphthalenesulfonate, basic aluminum iodide, zirconium salts, aluminum-zirconium composite salt, and zinc p-phenolsulfonate. These antiperspirants may be used alone or in combination of two or more.

Examples of the antimicrobial agents include nonionic antimicrobial agents such as 3,4,4-trichlorocarbanilide (TCC), resorcinol, phenol, hexachlorophene, triclosan and isopropylmethylphenol; cationic antimicrobial agents such as benzalkonium chloride, benzethonium chloride, alkyltrimethylammonium chloride and zinc pyrithione; and anionic antimicrobial agents such as sorbic acid and salicylic acid. These antimicrobial agents may be used alone or in combination of two or more.

Furthermore, both of said antiperspirants and antimicrobial agents may be used in combination. At least one of the antimicrobial agent is preferably used in the present invention.

The content of the antimicrobial agent, if used, is preferably from 0.01 to 5% by weight and more preferably from 0.05 to 3% by weight based on the liquid deodorant preparation. The content of the antiperspirant, if used, is preferably from 0.1 to 30% by weight and more preferably from 1.0 to 15% by weight based on the liquid deodorant preparation.

The component (B) of the liquid deodorant preparations is an unsaturated fatty acid preferably having from 14 to 22 carbon atoms and more preferably from 14 to 18 carbon atoms. Specific examples thereof include myristoleic acid, palmitoleic acid, oleic acid, linoleic acid and linolenic acid.

These unsaturated fatty acids may be used alone or in combination of two or more. The content of the unsaturated fatty acid in the liquid deodorant preparations is preferably from 0.01 to 1% by weight, more preferably from 0.02 to 0.5% by weight, and still more preferably from 0.05 to 0.3% by weight. Within the above range, the intended effects are obtained sufficiently. A content exceeding 1% by weight produces no noticeable improvement. A content of 1% by weight or less is preferred for product formulations, because the odor characteristic of the unsaturated fatty acid is not formed.

The liquid deodorant preparations contain the antioxidant as the component (C). Examples of the antioxidants include dibutylhydroxytoluene (BHT), butylhydroxyanisole, vitamin E and its derivatives, catechins, flavonoids, and polyphenols. These antioxidants may be used alone or in combination of two or mere.

The content of the antioxidant in the liquid deodorant preparations is selected from the range preferably from 0.001 to 0.2% by weight and more preferably from 0.005 to 0.1% by weight in view of the balance between long-lasting deodorant effect and production economy, etc.

The ratio of the contents of the component (C) and the component (B), (C)/(B), is preferably from 1/100 to 1/2 and more preferably from 1/50 to 1/5. If the ratio (C)/(B) is 1/100 or more, the long-lasting deodorant effect is enhanced sufficiently, while a ratio of 1/2 or less is preferred in view of production economy.

The components (A), (B) and (C) are dissolved or dispersed in the aqueous medium (D) to produce the liquid deodorant preparations of the present invention. The aqueous medium may include water and/or a water-soluble solvent such as lower alcohols mentioned below, with ethanol being particularly preferred.

By optionally incorporating a thickening agent as the component (E), the viscosity of the liquid deodorant preparation can be regulated within a suitable range for its use. The viscosity is preferably 30000 mPa·s or less, more preferably 20000 mPa·s or less and still more preferably 10000 mPa·s or less for its upper limit, and preferably 1000 mPa·s or more, more preferably 1500 mPa·s or more and still more preferably 2000 mPa-s or more for its lower limit. The viscosity is measured at 25°C using Brookfield viscometer. Namely, the viscosity is preferably from 1000 to 30000 mPa·s, more preferably from 1500 to 20000 mPa·s, and still more preferably from 2000 to 10000 mPa·s. Within the above range, the effect of reducing the running down of the liquid deodorant preparations upon their application to human skin is obtained.

The thickening agent (E) is a compound capable of regulating the viscosity of the liquid deodorant preparations within a desired level by dissolving in the aqueous medium (D), and may include water-soluble polymers. Examples thereof include poly(meth)acrylic acid, crosslinked poly(meth)acrylic acid, (meth)acrylic acid-C₁₋₃₀ alkyl (meth)acrylate copolymer, crosslinked (meth)acrylic acid-C₁₋₃₀ alkyl (meth)acrylate copolymer, cationic crosslinked copolymer, and further include hydroxyethylcellulose, hydroxypropylcellulose, carboxymethylcellulose, xanthan gum, carrageenan, gelatin, methylcellulose, ethylcellulose, polyvinyl alcohol, and their derivatives, with poly(meth)acrylic acid, (meth)acrylic acid- C₁₋₃₀ alkyl (meth)acrylate copolymer, crosslinked poly(meth)acrylic acid, crosslinked (meth)acrylic acid-C₁₋₃₀ alkyl (meth)acrylate copolymer, and cationic crosslinked copolymer being preferred.

The cationic crosslinked copolymer is a polymer having a cationic group and a crosslinked structure in its molecule, for example, a cationic crosslinked copolymer (copolymer X) produced by copolymerizing at least one cationic group-containing unsaturated monomer (monomer a1), at least one amide group-containing unsaturated monomer (monomer a2), and at least one crosslinkable unsaturated monomer having two or more unsaturated groups in its molecule (monomer a3).

Preferred examples of the monomers a1 include neutralized salts and quaternary ammonium salts each prepared from dimethylaminoethyl (meth)acrylate, diethylaminoethyl (meth)acrylate, dimethylaminopropyl (meth)acrylamide or diethylaminopropyl (meth)acrylamide by neutralizing its amino group with an acid or quatcrnizing its amino group with a quaternizing agent, and further include dimethyldiallylammonium chloride.

Preferred examples of the monomers a2 include N-methyl(meth)acrylamide, N,N-dimethyl(meth)acrylamide, N,N-diethyl(meth)acrylamide, N-n-propyl(meth)acrylamide, N-t-butylacryltimide, N-(meth)acryloylmorpholine, N-vinylpiperidone, and N-vinylpyrrolidone. Of these compounds, N,N-disubstituted acrylamides are preferred because the feeling upon the use of liquid deodorant preparations is good. More preferred is N,N-dimethyl(meth)acrylamide and N,N-diethyl(meth)acrylamide.

Examples of the monomers a3 include (meth)acrylate esters of polyhydrie alcohols or unsaturated alcohols, bis(meth)acrylamides, divinyl compounds, and polyallyl compounds. Preferred are ethylene glycol di(meth)acrylate, polyethylene glycol di(metb)acrylate, pentaerythritol tetra(meth)acrylate, pentaerythritol allyl ethers, vinyl (meth)acrylate, and allyl (meth)acrylate.

Preferred cationic crosslinked copolymers are copolymers produced from at least one monomer a1, at least one monomer a2 and at least one monomer a3, each selected from preferred examples recited above. Particularly, copolymers of dimethylaminoethyl (meth)acrylate/N,N-dimethyl(meth)acrylamide/polyethylene glycol di(meth)acrylate are preferred in view of obtaining a suitable viscosity.

The content of the monomer a3 is preferably from 0.002 to 5% by weight, more preferably from 0.002 to 3% by weight, and still more preferably from 0.002 to 1% by weight. Within the above range, the hydro gel formed by using the copolymer X exhibits a soft feeling and a good lubricity,

In addition to the constitutional monomer units derived from at least one monomer a1, at least one monomer a2 and at least one monomer a3, the copolymer X may further include a monomer unit derived from another kind of unsaturated monomer copolymerizable with these monomers, for example, (meth)acrylate ester monomers such as methyl (meth)acrylate and ethyl (meth)acrylate; anionic group-containing monomers such as acrylic acid and methacrylic acid; and betaines such as N-(3-sulfopropyl)-N-acrylyloyloxyethyl-N,N-dimethylammonium betaine and N-carboxymethyl-N-methacrylyloyloxyethyl-N,N-dimethylammonium betaine.

The copolymer X is produced, for example, by radical polymerization in any manner of aqueous solution polymerization, reverse-phase suspension polymerization, precipitation polymerization, etc.

The poly(meth)acrylic acid is a polymer of (meth)acrylic acid (monomer b1). The (meth)acrylic acid-C₁₋₃₀ alkyl (meth)acrylate copolymer is a copolymer of at least one monomer b1 and at least one C₁₋₃₀ alkyl (meth)acrylate (monomer b2). The crosslinked poly(meth)acrylic acid is a copolymer obtained by the copolymerization of at least one monomer b1 and at least one monomer a3 as the essential monomers. The crosslinked (meth)acrylic acid-C_{1·30} alkyl (meth)acrylate copolymer is a copolymer of at least one monomer b1, at least one monomer b2 and at least one monomer a3, wherein the content of the monomer a3 is preferably from 0.00001 to 5% by weight, more preferably from 0.00002 to 3% by weight, and still more preferably from 0.00005 to 1% by weight, each based on the total amount of the monomers.

When any of these (meth)acrylic acid-based polymers is used, it is preferred for obtaining a suitable viscosity to control the pH of the liquid deodorant preparations by a neutralizing agent, for example, inorganic bases such as sodium hydroxide and potassium hydroxide; alkanolamines such as monoethanolamine, diethanolamine, triethanolamine and aminomethylpropanol; and basic amino acids such as lysine, arginine and histidine.

The thickening agents as the component (E) may be used alone or in combination of two or more in an amount enough to regulate the viscosity of the liquid deodorant preparations within the range of from 1000 to 30000 mPa·s at 25°C, preferably from 0.01 to 10% by weight and more preferably from 0.05 to 5% by weight based on the total ingredients.

The viscosity of the liquid deodorant preparation is also regulated by incorporating a volatile solvent such as water; lower alcohols having from 1 to 4 carbon atoms; cyclic dialkylpolysiloxanes with a polymerization degree of from 4 to 5, particularly cyclic dimethylpolysiloxanes (for example, "Silicone SH-344," Dow Corning Toray Silicone Co., Ltd.); linear dialkylpolysiloxanes with a polymerization degree of from 2 to 5, particularly linear dimethylpolysiloxanes (for example, "Silicone KF-96A (2 cs)," Shin-Etsu Chemical Co., Ltd.); and light isoparaffin (for example, "Nisseki Isosol 300," Nippon Petrochemicals Company, Limited), with water and water-soluble solvents being preferred. Particularly preferred are water/lower alcohol mixtures in a mixing ratio of preferably from 1/99 to 50/50, more preferably from 10/90 to 50/50, and still more preferably from 20/80 to 45/55, each in terms of weight. By controlling the water/lower alcohol ratio within the above range, the quick-drying is promoted, a silky feeling is provided more easily when a powder, for example, is used in combination. The lower alcohol is preferably ethanol or isopropanol, and more preferably ethanol. The content of the volatile solvent in the liquid deodorant preparations, if used, is preferably from 40 to 99% by weight, more preferably from 60 to 99% by weight, and still more preferably from 80 to 99% by weight. The aqueous medium (D) described above also plays a role of regulating the viscosity.

To provide a more silky skin feeling, a powder as component (F) other than the component (A) may be further incorporated into the liquid deodorant preparations. Examples thereof include organic powders such as silicone resins (for example, "KMP-590" manufactured by Shin-Etsu Chemical Co., Ltd., "Tospearl® 145" and "Tospearl® 2000B" each manufactured by GE Toshiba Silicones, and "Trefil®" manufactured by Dow Corning Toray Silicone Co., Ltd.), nylon resins (for example, "SP-500" manufactured by Toray Industries Inc.), polystyrene resins (for example, "Finepearl" manufactured by Sumitomo Chemical Co., Ltd., "Techpolymer SB" manufactured by Sckisui Plastics Co., Ltd. and "Fine Powder SGP" manufactured by Soken Chemical & Engineering Co. Ltd.), polyethylene resins (for example, "Flo-Beads®" manufactured by Sumitomo Seika Chemicals Co., Ltd.), polymethyl methacrylate resins (for example, "Matsumoto Microsphere® M" manufactured by Matsumoto Yushi-Seiyaku Co., Ltd., "Techpolymer MB" manufactured by Sekisui Plastics Co., Ltd. and "Fine Powder MP" manufactured by Soken Chemical & Engineering Co., Ltd.), divinylbenzene resins, polyurethane resins, benzoguanamine resins, melamine resins, phenol resins, fluorine-containing resins and fine polymer particles prepared by the dispersion polymerization of vinyl monomer in a solvent in the presence of a dispersing agent, for example, a polysiloxane compound monoterminated with a radical-polymerizable group (JP 11-181003A); and inorganic powders such as talc, sericite, mica, kaolin, red iron oxide, clay, bentonite, silicic anhydride, and synthetic silica beads.

The shape of powder may be any of sphere, column, plate and needle, with the spherical shape being preferred in view of its smoothness. Although the sphericity of powder is not critical, a powder of nearly complete sphere is preferably used because the coefficient of dynamic friction is reduced as the sphericity increases, thereby to provide a more smooth skin feeling.

The average particle size of powder is preferably from 0.05 to 50 µm and more preferably from 0.5 to 50 µm when measure by a laser diffraction/scattering method.

The powder may be subjected to a hydrophobic treatment such as silicone treatment, fluorine treatment, metallic soap treatment, and fatty acid treatment.

Two or more kinds of powders may be used. The content of the powder in the liquid deodorant preparations, if used, is preferably from 0.1 to 40% by weight, more preferably from 1 to 20% by weight, and still more preferably from 2 to 8% by weight, in view of obtaining a sufficient effect in improving skin feeling and in preventing the formation of white residues after application to human skin.

By incorporating an oily component, the relative dynamic friction coefficient of the liquid deodorant preparations can be regulated so as to provide a desired silky feeling. Examples of the oily components include silicone oils such as dimethylpolysiloxane, dimethylcyclopolysiloxane, and methylhydrogenpolysiloxane; hydrocarbons such as solid or liquid paraffin, crystal oil, ceresin, ozokerite, and montan wax; vegetable or animal fats and oils such as olive oil, mineral wax, carnauba wax, lanolin, and whale wax; fatty acids such as stearic acid, palmitic acid, and oleic acid; fatty acid esters such as glycerol monostearate, glycerol distearate, glycerol monooleate, isopropyl myristate, isopropyl stearate, butyl stearate, and neopentyl glycol dicaprate; and higher alcohols such as cetyl alcohol, stearyl alcohol, palmityl alcohol, and hexyldodecyl alcohol, with isopropyl myristate being preferred.

The content of the oily component in the liquid deodorant preparations, if used, is preferably from 0.05 to 10% by weight and more preferably from 0.1 to 5% by weight.

The liquid deodorant preparations of the present invention may be used in the form of sheet cosmetic products which are prepared by impregnating a sheet substrate with the liquid deodorant preparation. The amount of the liquid deodorant preparation supported in the sheet cosmetic product is preferably from 50 to 500 parts by weight and more preferably 100 to 400 parts by weight in view of obtaining a better feeling upon its use, each based on 100 parts by weight of the sheet substrate. The sheet substrate usable is any of nonwoven fabrics or woven fabrics each made of natural fibers or synthetic fibers. The liquid deodorant preparation is made supported in the sheet substrate by a spraying method using sprayer or air gun, an application method using slit nozzle or bar coater, and other methods.

The following examples further describe and demonstrate embodiments of the present invention.

### PRODUCTION EXAMPLE 1

A mixture of 50.6 g of N,N-dimethylaminoethyl methacrylate diethylsulfate ("MOEDES" manufactured by Nitto Chemical Co., Ltd.; 80% by weight aqueous solution), 51.55 g of N,N-dimethylacrylamide, 0.096 g of polyoxyethylene(14) glycol dimethacrylate ("NK-14G" manufactured by Shin· Nakamura Chemical Co.,Ltd.) and 350 g of ion-exchanged water was purged with nitrogen gas, to prepare an aqueous solution of monomers. After charging the aqueous solution of monomers into a reaction vessel, the temperature was raised to 55°C while replacing the atmosphere of reaction system with nitrogen by flowing it for 20 min. Then, 0.19 g of 2,2'-azobis(2-amidinopropane) dihydrogenchloride was added as an polymerization initiator. The polymerization started after 30 min of the addition, and the whole reaction system changed to a soft gel-like state. The stirring was further continued, and the polymerization was stopped after 4 hrs of the addition of polymerization initiator. The resultant doughy product was taken out, washed in 5-L ethanol under stirring for 10 min and then dried. The dried product was crushed by a jet mill, to obtain a copolymer X.

### EXAMPLES 1-4 and COMPARATIVE EXAMPLES 1-2

Each deodorant preparation shown in Table 1 was prepared and a silky skin feeling, a dry feeling of deodorant preparation and an irritant feeling due to solvent were evaluated according to the following criteria. The results arc shown in Table 1.

### Evaluation of Dry Feeling, Silky Skin Feeling and Irritant Feeling

Each deodorant preparation was applied to upper arms of ten panelists, and then the dry feeling, the silky skin feeling and the irritant feel were evaluated according to the following scores.

### Evaluation Scores

(1) Dry feeling
   4: perceived very quickly.
   3: perceived quickly.
   2: perceived not so much.
   1: not perceived.
(2) Silky skin feeling
   4: very silky.
   3: silky.
   2: not so silky.
   1: not silky.
(3) Irritant feeling
   4: not irritant.
   3: slightly irritant.
   2: somewhat irritant.
   1: very irritant.

### Averaged Evaluation Sores

3.5 to 4.0: ⊚
2.5 to 3.4: ○
1.5 to 2.4: Δ
1.0 to 1.4: ×

### Measurement of Viscosity

The viscosity of each deodorant preparation was measured at 25°C using Brookfield viscometer under the conditions shown in Table 1.

**Table 1**

| | Examples | | | | Comparative Examples | |
|---|---|---|---|---|---|---|
| | 1 | 2 | 3 | 4 | 1 | 2 |
| Ingredients (% by weight) | | | | | | |
| Component (A) | | | | | | |
| isopropylmethylphenol | 0.1 | 0.1 | 0.1 | 0.1 | - | 0.1 |
| aluminum chlorohydrate | - | - | 5 | - | - | - |

| Component (B) | | | | | | |
|---|---|---|---|---|---|---|
| oleic acid | 0.2 | 0.1 | 0.05 | 0.2 | - | - |
| linoleic acid | - | 0.05 | - | - | - | - |
| linolenic acid | - | 0.05 | - | - | - | - |

| Component (C) | | | | | | |
|---|---|---|---|---|---|---|
| BHT | 0.02 | 0.02 | 0.02 | 0.02 | 0.02 | 0.02 |

| Component (D) | | | | | | |
|---|---|---|---|---|---|---|
| ethanol | 70 | 70 | 70 | 50 | 25 | 70 |
| water | bal. | bal. | bal. | bal. | bal. | bal. |

| Component (E) | | | | | | |
|---|---|---|---|---|---|---|
| (meth)acrylic copolymer*¹ | 0.3 | - | - | 0.3 | 0.3 | 0.3 |
| copolymer X*² | - | 0.3 | - | - | - | - |

| Component (F) | | | | | | |
|---|---|---|---|---|---|---|
| silicone powder*³ | 5 | 5 | - | 5 | 5 | 5 |

| Other Components | | | | | | |
|---|---|---|---|---|---|---|
| triethanolamine | 0.075 | - | - | 0.075 | 0.075 | 0.075 |
| Total | 100 | 100 | 100 | 100 | 100 | 100 |
| (C)/(B) | 1/10 | 1/10 | 1/2.5 | 1/10 | - | - |

| Evaluation Results | | | | | | |
|---|---|---|---|---|---|---|
| dry feeling | ⊚ | ⊚ | ⊚ | ○ | × | ⊚ |
| silky feeling | ⊚ | ⊚ | ○ | ⊚ | ○ | ⊚ |
| irritant feeling due to solvent | ○ | ○ | ○ ⊚ △ | | | △ |

| Measurement of Viscosity | | | | | | |
|---|---|---|---|---|---|---|
| viscosity (mPa·s) | 8500 | 2800 | 4.2 | 8800 | 28600 | 20200 |
| spindle number | 4 | 4 | 1 | 4 | 4 | 4 |
| rotation speed (rpm) | 6 | 6 | 60 | 6 | 6 | 6 |

| | | | | | | |
|---|---|---|---|---|---|---|
| *¹: (meth)acrylic acid-C₁₀₋₃₀ alkyl (meth)acrylate copolymer ("Carbopol ETD2020" manufactured by Noveon Inc.). | | | | | | |
| *²: prepared in Production Example 1. | | | | | | |
| *³: methicone silsesquioxane ("KMP-590" manufactured by Shin-Etsu Chemical Co., Ltd.). | | | | | | |

### EXAMPLE 5

The following liquid deodorant preparation was prepared, which dried quickly after applied to human skin and was excellent in the silky skin feeling and the dry feeling of the liquid deodorant preparation. The viscosity of the deodorant preparation measured at 25°C using Brookfield viscometer was 1050 mPa-s (number 3 spindle, 12 rpm). The liquid deodorant preparation was also easy to handle without spilling from hand upon its application and less irritant to human skin.

| | |
|---|---|
| Liquid Deodorant Preparation (% by weight) | |
| Triclosan: | 0.2 |
| Nylon powder*⁴: | 20.0 |
| Ethanol: | 40.0 |
| Cross-linked polyacrylic acid*⁵: | 0.3 |
| Oleic acid: | 0.1 |
| Linoleic acid: | 0.05 |
| Linolenic acid: | 0.05 |
| Triethanolamine: | 0.3 |
| Purified water: | balance for adding up to 100 percent by weight. |

| | |
|---|---|
| *⁴: "Nylon Powder SP-500" manufactured by Toray Industries. | |
| *⁶: "Carbopol 940" manufactured by Noveon Inc. | |

### INDUSTRIAL APPLICABILITY

The liquid deodorant preparations of the present invention dry quickly on human skin, provide a good silky skin feeling with no wet feeling, is easy to handle without spilling from hand upon its application, and is less irritant to human skin.

## Claims

1. A liquid deodorant preparation comprising (A) an antiperspirant and/or an antimicrobial agent, (B) an unsaturated fatty acid, (C) an antioxidant, and (D) an aqueous medium.

2. The liquid deodorant preparation according to claim 1, wherein the unsaturated fatty acid (B) is at least one compound selected from myristoleic acid, palmitoleic acid, oleic acid, linoleic acid and linolenic acid.

3. The liquid deodorant preparation according to claim 1 or 2, wherein the aqueous medium (D) is a water/lower alcohol mixture in a ratio of from 1/99 to 50/50 by weight.

4. The liquid deodorant preparation according to any one of claims 1 to 3, which further comprises (E) a thickening agent.

5. The liquid deodorant preparation according to any one of claims 1 to 4, which further comprises a powder other than the component (A).

6. The liquid deodorant preparation according to any one of claims 1 to 5, wherein the content of the component (B) is from 0.01 to 1 % by weight.

7. The liquid deodorant preparation according to any one of claims 1 to 6, wherein the content of the component (C) is from 0.001 to 0.2 % by weight.

8. The liquid deodorant preparation according to any one of claims 1 to 7, wherein the content ratio of the component (C) and the component (B), (C)/(B), is from 1/100 to 1/2.

9. Use of a composition comprising (A) an antiperspirant and/or an antimicrobial agent, (B) an unsaturated fatty acid, (C) an antioxidant, and (D) an aqueous medium as a liquid deodorant preparation.
